# EUROPÄISCHE PATENTANMELDUNG

(11) **EP 1 405 631 A1**
(43) Veröffentlichungstag der Anmeldung: **07.04.2004**
(21) Anmeldenummer: 03019722.2
(22) Anmeldetag: 29.08.2003
(51) Int. Cl.: A61K 7/48

(54) **Kosmetische Formulierung**

(30) Priorität: 18.09.2002 DE 10243437
(71) Anmelder: PM-International AG, 1618 Luxembourg (LU)
(72) Erfinder: Schmitt, Gerhard, Dr., 64625 Bersheim (DE)
(74) Vertreter: Zech, Stefan Markus Dipl.-Phys.

(57) **Zusammenfassung**

Die Erfindung betrifft eine kosmetische Formulierungen zur dermatologischen Anwendung enthaltend mindestens eine fettlösliche Vitaminkomponente sowie mindestens eine wasserlösliche Vitaminkomponente, die dadurch gekennzeichnet ist, dass zumindest die mindestens eine fettlösliche Vitaminkomponente in micellierter Form vorliegt, und wobei die mindestens eine fettlösliche Vitaminkomponente das Coenzym Q10 umfasst.

## Beschreibung

Die Erfindung betrifft eine kosmetische Formulierung zur dermatologischen Anwendung.

Um eine pflegende, schützende oder straffende Wirkung für die Haut zu erzielen ist es bekannt kosmetischen Formulierungen unterschiedliche kosmetische Wirkstoffkomponenten beizumischen. Diese können beispielsweise wasseroder fettlösliche Vitamine wie Vitamin C, Vitamin E, Vitamin A oder Coenzym Q10, Fette oder Öle oder weitere pflanzliche oder chemische Zusatzstoffe umfassen.

Bei kosmetischen Formulierungen besteht insbesondere hinsichtlich etwaig enthaltener Vitaminkomponenten aber auch hinsichtlich sonstiger Wirkstoffkomponenten das Problem, dass die einzelnen Vitamin- bzw. Wirkstoffkomponenten nicht ausreichend stabil sind und/oder nicht ausreichend am Wirkort resorbiert werden und/oder nicht weit genug in die Haut an den gewünschten Wirkort dringen.

Aus WO 94/13265 A1 ist eine Zusammensetzung zur Verminderung freier Radikale im Körper bekannt, die Liposome umfasst, in die wenigstens zwei der Antioxidantien Beta-Karotin, Vitamin C, Vitamin E, Glutathion oder Niacin und optional wenigstens ein Spurenelement eingebracht sind. Diese Zusammensetzung kann dem Körper über eine Creme oder Lotion oder als Medikament zum Beispiel oral, intravenös oder subkutan zugeführt werden. Die Liposome transportieren die Wirkstoffe im Körper, beispielweise in den Hautschichten.

Die Aufgabe der vorliegenden Erfindung besteht darin, eine kosmetische Formulierung zur dermatologischen Anwendung bereitzustellen, die insbesondere einer Hautalterung entgegenwirkt und bei der die entsprechenden Vitaminkomponenten bzw. Wirkstoffkomponenten am gewünschten Ort ihre Wirkung effektiv entfalten können.

Diese Aufgabe wird gelöst durch eine kosmetische Formulierung nach den Merkmalen des Anspruches 1. Vorteilhafte Weiterbildungen sind in den Unteransprüchen angegeben.

Die kosmetische Formulierung zur dermatologischen Anwendung gemäß Anspruch 1 enthält mindestens eine fettlösliche Vitaminkomponente sowie mindestens eine wasserlösliche Vitaminkomponente, wobei die mindestens eine fettlösliche Vitaminkomponente in micellierter Form vorliegt, wobei die mindestens eine wasserlösliche Vitaminkomponente als Solubilisat oder in micellierter Form vorliegt und wobei die mindestens eine fettlösliche Vitaminkomponente das Coenzym Q10 umfasst.

Derartige Micellen bzw. die Einbettung bestimmter Vitamine in Micellen ist an sich bekannt und wird vom menschlichen Körper selbst zur besseren Verwertung von Nährstoffen im intestinalen Bereich genutzt. Bei Einsatz von in micellierter Form vorliegenden fettlöslichen Vitaminkomponenten, insbesondere auch Coenzym Q10, in einer kosmetischen Formulierung hat sich gezeigt, dass dann diese fettlöslichen Vitaminkomponenten wesentlich besser von der Haut resorbiert werden und wesentlich effektiver an den vorgesehenen Wirkort gelangen. Untersuchungen haben ergeben, dass nicht nur ein schnelleres, sondern auch ein tieferes Eindringen der fettlöslichen Vitaminkomponenten auf diese Weise erzielbar ist. Gleichzeitig schützt die Micellenstruktur davor, dass die fettlöslichen Vitaminkomponenten vor ihrer Anwendung in ihrer Struktur verändert werden. Dasselbe gilt auch für die wasserlöslichen Vitaminkomponenten, die zusammen mit den fettlöslichen Vitaminkomponenten oder auch separat in einer Micelle vorliegen können.

Besonders vorteilhaft kann es jedoch bei wasserlöslichen Vitaminkomponenten sein, beispielsweise bei Vitamin C oder den entsprechenden Derivaten, wenn sie nicht in einer Micelle eingebettet sind, sondern als Solubilisat vorliegen. Die betreffende Vitaminkomponente ist dann vor vorzeitigen Reaktionen bzw. Veränderungen, insbesondere vor vorzeitiger Oxidation geschützt.

Speziell Vitamin C, bzw. Ascorbinsäure (ASC) ist Dank ihrer labilen En-diol Struktur ein ausgezeichnetes Reduktionsmittel im biologischen Redoxbereich, in Anwesenheit von Übergangsmetallionen (Fe, Cu) jedoch auch ein instabiles Prooxidans. Biologisch "sicher" ist Ascorbat nur in Anwesenheit kooperativ wirkender Helfermoleküle wie Tokopherol oder bestimmter Flavonoide wie Rutin oder Quercetin mit ihren o-Dihydroxy-Äquivalenten. Damit vermittelt die Ascorbinsäure synergistisch mit dem Tokopherol zwischen Reduktionsäquivalenten im wässrigen Milieu und der Lipidphase, wo zum Beispiel die Karotinoide als Antioxidantien ansetzen. Die Stabilität der Ascorbinsäure ist nicht nur von der An- oder Abwesenheit von Übergangsmetallionen abhängig, sondern auch stark vom jeweiligen pH-Wert. Sie autooxidiert im basischen Milieu, wobei dem Anschein nach auch extrem niedrige Übergangsmetallkonzentrationen, die im menschlichen Körper stets vorhanden sind, katalytisch wirken. Die Untersuchung der Stabilitätskinetik von wässriger ASC im Vergleich zum ASC-Solubilisat ergibt die bereits erwähnte Abhängigkeit vom pH-Wert. Nach 200 Minuten sind bei pH 3,7 ca. 20 % oxidiert, bei pH 7,0 ca. 50 % und bei pH 9,0 ca. 65 %.

Besonders bei basischem pH ist ersichtlich, dass dieser Abbau biphasisch verläuft. Der Abbau der ASC wurde mit Dichlorphenol-Indophenol (DCPIP) bestimmt, welches durch ASC seine blaue Farbei verliert und zur "Leukobase" reduziert wird. Dies wird ersichtlich durch den Absorptionsverlust bei 600 nm, wobei sich auch eine Verschiebung des Absorptionsmaximums nach 515 nm ergibt.

Obgleich sich in der Initialkinetik der Autooxidation keine signifikanten Unterschiede zwischen ASC-wässrig und ASC-Solubilisat erkennen lassen, ergeben diese sich in markanter Form in der Langzeitwirkung: während wässrige ASC nach 3240 Minuten die Reoxidation des entfärbten DCPIP mit dem ursprünglichen Absorptionsverhalten (max. Absorption bei 600 nm) zulässt, behält das ASC-Solubilisat den reduziereten Zustand des DCPIP aufrecht und erlaubt keine Reoxidation an der Luft. Dies ist jedoch nur bei pH-Werten im neutralen Bereich und noch besser im leicht sauren Milieu der Fall; im Basischen (pH 9,0) lässt sich zwischen ASC-wässrig und ASC-Solubilisat kein markanter Unterschied erkennen.

Als Vitaminkomponenten im Sinne der vorliegenden Erfindung werden alle Vitamine sowie vitaminähnliche Substanzen, insbesondere auch vitaminoide Substanzen angesehen. Konkret kann die mindestens eine Vitaminkomponente zusätzlich die Vitamine A und/oder E umfassen. Alternativ oder zusätzlich kann die fettlösliche Vitaminkomponente Retinol umfassen.

Nach einem weiteren Aspekt der vorliegenden Erfindung können neben der mindestens einen in micellierter Form vorliegenden Vitaminkomponente andere kosmetische Wirkstoffkomponenten in der kosmetischen Formulierung enthalten sein. Es hat sich gezeigt, dass die in micellierter Form vorliegenden fettlöslichen Vitaminkomponenten mit der wesentlich erhöhten Resorbierbarkeit dieser Komponenten durch die Haut bei Auftragen bzw. Einwirken der kosmetischen Formulierung eine Art "Mitreißeffekt" erzeugen, der auch ein tieferes Eindringen weiterer Wirkstoffkomponenten hervorruft. Durch diesen "Mitreißeffekt" können auch andere, insbesondere nicht in micellierter Form vorliegende Wirkstoffkomponenten wesentlich effektiver, insbesondere schneller und/oder tiefer innerhalb der Haut resorbiert werden.

In einer konkret bevorzugten Ausgestaltung umfasst die wasserlösliche Vitaminkomponente das Vitamin C oder Derivate hiervon.

Nach einem bevorzugten Aspekt der vorliegenden Erfindung umfassen die kosmetischen Wirkstoffkomponenten Liposome, die zumindest teilweise auch Bestandteile der Micellen bilden. Die Liposome transportieren die Vitamine in tiefere Hautschichten und geben sie dort zur Wirkung frei.

Die kosmetischen Wirkstoffkomponenten können ergänzend oder alternativ Hyaluronsäure umfassen. Die Hyaluronsäure hat in der vorliegenden kosmetischen Formulierung den Zweck, Feuchtigkeit zu binden und die Haut vor Austrocknen zu bewahren. Damit wird gleichzeitig das Spannungsgefühl besonders beanspruchter Haut reduziert. Die Hyaluronsäure enthaltende kosmetische Formulierung wirkt damit beruhigend und verbessert die Hydrobalance. Hierdurch wird als Folgeerscheinung die natürliche Abwehrfunktion der Haut unters tützt.

Anstelle oder zusätzlich zur Hyaluronsäure kann auch ein anderer Feuchtigkeitskomplex zum Einsatz gelangen.

Die kosmetische Formulierung kann ergänzend oder alternativ Bisabolol und/oder Allantoin als weitere kosmetische Wirkstoffkomponente umfassen.

Die genannten Wirkstoffkomponenten wirken entzündungshemmend und beruhigend. Sie aktivieren die Zellfunktion und unterstützen die natürliche Hautbalance (Durchblutung, pH-Wert, Feuchtigkeitsgehalt). Durch eine beschleunigte Zellerneuerung wird ein Anti-Aging-Effekt unterstützt.

In einer besonders bevorzugten Ausgestaltung sind die mindestens eine in micellierter Form vorliegende Vitaminkomponente und die mindestens eine weitere kosmetische Wirkstoffkomponente so aufeinander abgestimmt, dass die kosmetische Wirkstoffkomponente durch die in micellierter Form vorliegende Vitaminkomponente bei dermatologischer Anwendung in tiefere Hautschichten mitgerissen wird. Es ergibt sich dadurch ein synergistischer Effekt, der nicht nur eine verbesserte Wirkung der Vitaminkomponente, sondern auch eine verbesserte Wirkung der weiteren Wirkstoffkomponenten durch bessere Resorption in der Haut mit sich bringt.

Die kosmetische Formulierung kann zusammen mit weiteren Grund- oder Zusatzstoffen als Creme, insbesondere als 24-Stunden-, Nacht-, Tages-, Bodyoder Augencreme vorliegen, wobei sich die genaue Zusammensetzung der kosmetischen Formulierung je nach bevorzugter Wirkung unterscheiden kann. Diesbezügliche Ausführungsbeispiele werden weiter unten noch angegeben.

Die kosmetische Formulierung kann zusammen mit weiteren Grund- oder Zusatzstoffen auch als Reinigungsmilch, als Pflegeemulsion, Körperlotion oder als Maske, insbesondere Feuchtigkeitsmaske vorliegen.

Die verschiedenen Vitamin- und Wirkstoffkomponenten der kosmetischen Formulierung bilden insgesamt ein Wirkstoffgruppenpräparat, bei dem die Einzelkomponenten in synergistischer Weise ineinandergreifen. Des weiteren wird das in der Ernährungswissenschaft angewandte Nährstofftransportkonzept entsprechend in ein Wirkstofftransportkonzept übertragen, dergestalt, dass in der kosmetischen Formulierung nicht nur die Vitamin- und Wirkstoffkomponenten selbst, sondern auch entsprechende Transportmittel vorgesehen werden, die einen Antransport an den vorgesehenen Wirkort gewährleisten. Dies wird im vorliegenden Fall durch die Micellen-Struktur erreicht, die einerseits einen Schutz der Vitaminkomponenten, eine bessere Resorptionsfähigkeit und/oder eine erhöhte Eindringtiefe bewirken. Gleichzeitig entsteht ein "Mitreißeffekt", der andere micellierte Vitamin- oder Wirkstoffkomponenten sowie auch nichtmicellierte Vitamin- oder Wirkstoffkomponenten mitreißt.

In der nachfolgenden Tabelle ist eine kosmetische Pflegeserie dargestellt, bei der die Hauptkomponenten der Pflegeserie auf die kosmetische Formulierung nach der Erfindung zurückgreifen und die durch einige ergänzende Produkte abgerundet wird. Wie aus der Tabelle ersichtlich, werden die Vitamin- und Wirkstoffkomponenten je nach konkreten Pflegeprodukt vorgesehen, weggelassen oder kombiniert. Bei der Augencreme, Nachtcreme oder Tagescreme nach dem vorliegenden Ausführungsbeispiel sind beispielsweise sowohl Vitamin E als auch Q10 vorgesehen. Eine allgemeine Erkenntnis der vorliegenden Erfindung besteht auch darin, dass sich innerhalb einer kosmetischen Formulierung zur dermatologischen Anwendung Q10 und Vitamin E gegenseitig unterstützen, und zwar insbesondere dadurch, dass sie ihre Funktion als ElektronenDonatoren zum Abfangen freier Radikale regenerieren.

Im Einzelnen sind bei der Pflegeserie nach dem vorliegenden Ausführungsbeispiel in der Pflegeemulsion die Vitaminkomponente Vitamin E sowie als Wirkstoffkomponente ein Feuchtigkeitskomplex nach dem Stand der Technik vorgesehen. In der Feuchtigkeitsmaske der vorliegenden Ausführungsform ist Q10 in micellierter Form, ein Feuchtigkeitskomplex, Allantoin sowie Bisabolol enthalten. Die bereits angesprochene Augencreme nach der vorliegenden Ausführungsform umfasst Vitamin E, Retinol, Vitamin C in micellierter Form sowie Q10 ebenfalls in micellierter Form. Die Nachtcreme der vorliegenden Ausführungsform umfasst Vitamin E, Retinol, Q10 (in micellierter Form) sowie Allantoin. Die Tagescreme der vorliegenden Ausführungsform umfasst Vitamin E, einen UV-Protektor, Q10 (in micellierter Form), einen Feuchtigkeitskomplex nach dem Stand der Technik sowie Hyaluronsäure. Die Körpercreme der vorliegenden Ausführungsform umfasst Vitamin E, Retinol sowie Allantoin. In der Körperlotion der vorliegenden Ausführungsform ist Vitamin E, Aloe Vera sowie Menthol vorgesehen. In der 24-Stunden-Creme der vorliegenden Ausführungsform ist ein UV-Protektor, Vitamin C (in micellierter Form), ein Feuchtigkeitskomplex nach dem Stand der Technik sowie Bisabolol enthalten.

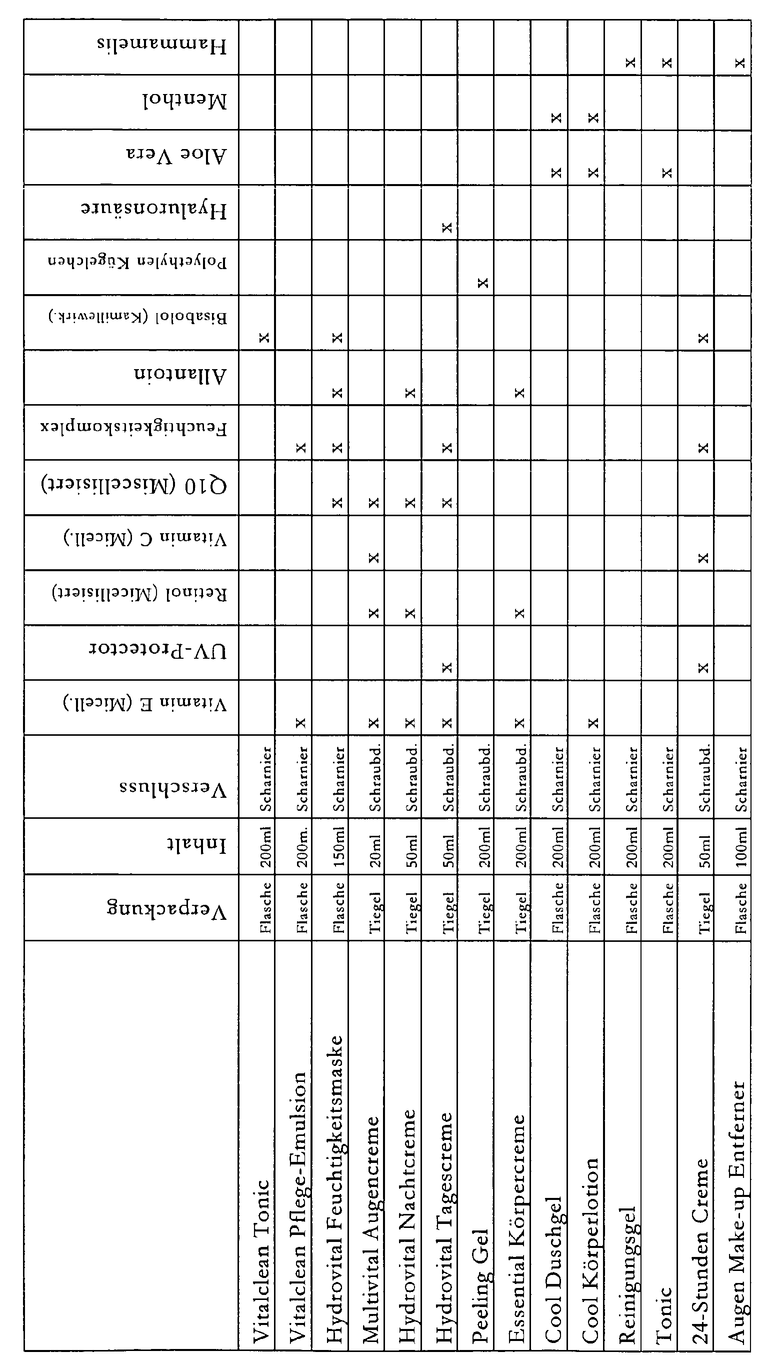

## Patentansprüche

**1.** Kosmetische Formulierung zur dermatologischen Anwendung, enthaltend mindestens eine fettlösliche Vitaminkomponente sowie mindestens eine wasserlösliche Vitaminkomponente,
wobei die mindestens eine fettlösliche Vitaminkomponente in micellierter Form vorliegt,wobei die mindestens eine wasserlösliche Vitaminkomponente als Solubilisat oder in micellierter Form vorliegt und wobei die mindestens eine fettlösliche Vitaminkomponente das Coenzym Q 10 umfasst.

**2.** Kosmetische Formulierung nach Anspruch 1,
**dadurch gekennzeichnet,**
**dass** die mindestens eine fettlösliche Vitaminkomponente Vitamin A und/oder Vitamin E umfasst.

**3.** Kosmetische Formulierung nach einem der Ansprüche 1 oder 2,
**dadurch gekennzeichnet,**
**dass** die mindestens eine fettlösliche Vitaminkomponente Retinol umfasst.

**4.** Kosmetische Formulierung nach einem der Ansprüche 1 bis 3,
**dadurch gekennzeichnet,**
**dass** neben der mindestens einen in micellierter Form vorliegenden Vitaminkomponente andere kosmetische Wirkstoffkomponenten enthalten sind.

**5.** Kosmetische Formulierung nach einem der Ansprüche 1 bis 4,
**dadurch gekennzeichnet,**
**dass** die mindestens eine wasserlösliche Vitaminkomponente Vitamin C umfasst.

**5.** Kosmetische Formulierung nach einem der Ansprüche 1 bis 5,
**dadurch gekennzeichnet,**
**dass** die kosmetischen Wirkstoffkomponenten Liposome umfassen.

**7.** Kosmetische Formulierung nach einem der Ansprüche 1 bis 6,
**dadurch gekennzeichnet,**
**dass** die kosmetischen Wirkstoffkomponenten Hyaluronsäure umfassen.

**8.** Kosmetische Formulierung nach einem der Ansprüche 1 bis 7,
**dadurch gekennzeichnet,**
**dass** die kosmetischen Wirkstoffkomponenten Bisabolol und/oder Allantoin umfassen.

**9.** Kosmetische Formulierung nach einem der Ansprüche 1 bis 8,
**dadurch gekennzeichnet,**
**dass** die mindestens eine in micellierter Form vorliegende Vitaminkomponente und die mindestens eine weitere kosmetische Wirkstoffkomponente so aufeinander abgestimmt sind, dass die kosmetische Wirkstoffkomponente durch die in micellierter Form vorliegende Vitaminkomponente bei dermatologischer Anwendung durch die in micellierter Form vorliegende Vitaminkomponente in tiefere Hautschichten mitgerissen wird.

**10.** Kosmetische Formulierung nach einem der Ansprüche 1 bis 9,
**dadurch gekennzeichnet,**
**dass** die kosmetische Formulierung zusammen mit weiteren Grundund/oder Zusatzstoffen als Creme, insbesondere 24-Stunden-, Nacht-, Tages-, Body- oder Augencreme vorliegt.

**11.** Kosmetische Formulierung nach einem der Ansprüche 1 bis 10,
**dadurch gekennzeichnet,**
**dass** die kosmetische Formulierung zusammen mit weiteren Grundoder Zusatzstoffen als Pflegeemulsion, Maske, insbesondere Feuchtigkeitsmaske oder Körperlotion vorliegt.
